# EUROPEAN PATENT APPLICATION

(11) **EP 4 269 586 A1**
(43) Date of publication of application: **01.11.2023**
(21) Application number: 22170992.6
(22) Date of filing: 29.04.2022
(51) Int. Cl.: C12N 15/113, A61P 9/12, A61P 9/04

(54) **TARGETING MICRO RNA FOR TREATMENT OF HEART FAILURE WITH PRESERVED EJECTION FRACTION (HFPEF)**

(71) Applicant: Johann-Wolfgang-Goethe-Universität Frankfurt am Main, 60323 Frankfurt am Main (DE)
(72) Inventor: DIMMELER, Stefanie, 60594 Frankfurt am Main (DE); ZEIHER, Andreas, 60594 Frankfurt am Main (DE); FISCHER, Ariane, 60388 Frankfurt am Main (DE)
(74) Representative: Kuttenkeuler, David

(57) **Abstract**

The invention pertains to a compounds or compositions comprising antagonists of miR-92 which are useful in a method of treatment of a heart disease associated with or caused by a reduced ventricular elasticity and/or a diastolic dysfunction in a subject, which preferably is a subject suffering from Heart Failure with preserved Ejection Fraction (HFpEF). The invention provides such compounds and/or compositions, which are preferably nucleic acid based antagonists of miR-92, preferably oligonucleotide inhibitors, as well as uses and method for treating subjects.

## Description

### FIELD OF THE INVENTION

The invention pertains to a compounds or compositions comprising antagonists of miR-92 which are useful in a method of treatment of a heart disease associated with or caused by a reduced ventricular elasticity and/or a diastolic dysfunction in a subject, which preferably is a subject suffering from Heart Failure with preserved Ejection Fraction (HFpEF). The invention provides such compounds and/or compositions, which are preferably nucleic acid-based antagonists of miR-92, preferably oligonucleotide inhibitors, as well as uses and method for treating subjects.

### DESCRIPTION

Half of patients with heart failure (HF) have a preserved left ventricular ejection fraction. Sometimes referred to as diastolic HF, heart failure with preserved ejection fraction (HFpEF) represents ^{~}50% of heart failure worldwide. Disability and frequent hospitalization are hallmarks of the disease. Associated comorbidities are common and notably include hypertension, diabetes, and obesity. Women are affected more frequently than men (by as much as a 2:1 preponderance). Unlike heart failure with reduced ejection fraction (HFrEF), where numerous pharmacological and device options have been proven to be effective, no treatments have been proven to reduce morbidity and mortality in HFpEF. The challenge of HFpEF is increasing as the population ages and comorbidities become more prevalent. The HFpEF hospitalization rate is now greater than that for heart failure with HFrEF. Compounding the problem is the fact that underlying pathophysiological mechanisms have not been completely elucidated.

Morbidity and mortality in HFpEF are similar to values observed in patients with HF and reduced ejection fraction (EF). However, HFpEF constitutes a distinct clinical syndrome refractory to routine medical approaches. No effective treatment has been identified and HFpEF has become a major public health concern. Its increasing prevalence, rising at a rate of ^{∼}1% per year, now exceeds that of heart failure with reduced ejection fraction (HFrEF). Outcomes of HFpEF are poor, and, so far, no treatment has been shown to decrease morbidity or mortality. For example, treatment to date has focused on the renin-angiotensin-aldosterone system and the adrenergic nervous system, but clinical trials have failed to show any significant benefit to their blockade. HFpEF, sometimes referred to as diastolic HF, is associated with various cardiovascular risk factors (especially hypertension), extra-cardiac comorbidities and aging. The net result is impaired diastolic relaxation and filling of the left ventricle (LV), increased myocardial stiffness, impaired vascular compliance, and increased diastolic pressure. Perhaps underlying different responses to pharmacological intervention, HFpEF populations can consist of patients with limited myocardial infarction at risk for unfavourable eccentric LV remodelling. Cardiac hypertrophy indeed has little in common with limited myocardial infarction, and in both conditions, mechanisms driving LV remodelling are likely to be dissimilar. There is a great need in the art for therapeutic approaches for HFpEF. Thus, it is an object of the invention.

MicroRNAs (miRNAs) are a class of small, endogenous and non-coding RNAs able to regulate posttranscriptional expression of target genes. MicroRNAs have been implicated in a number of biological processes including regulation and maintenance of cardiac function, vascular inflammation and development of vascular pathologies. Micro-RNA 92 (miR-92) has been implicated as a therapeutic target in the treatment of cardiovascular pathologies. Accordingly, modulating the function and/or activity of miR-92 may present as a therapeutic target in the development of effective treatments for particular types of heart failure and associated symptoms.

There is a need for microRNA-based treatments of heart failure such as HFpEF. The compositions and methods described herein address this need.

### BRIEF DESCRIPTION OF THE INVENTION

Generally, and by way of brief description, the main aspects of the present invention can be described as follows:

In **a first aspect,** the invention pertains to a micro-RNA (miR)-92 antagonist for use in the treatment or prevention of a heart disease in a subject, wherein the heart disease is caused by or associated with or caused by a reduced ventricular elasticity and/or a diastolic dysfunction.

In **a second aspect,** the invention pertains to a method of treating heart disease in a subject, the method comprising a step of administering a therapeutically effective amount of a micro-RNA (miR)-92 antagonist to the subject to treat the heart disease, and wherein the heart disease is caused by or associated with or caused by a reduced ventricular elasticity and/or a diastolic dysfunction.

In **a third aspect,** the invention pertains to the use of a micro-RNA (miR)-92 antagonist in the manufacture of a medicament for use in the treatment or prevention of a heart disease in a subject, wherein the heart disease is caused by or associated with or caused by a reduced ventricular elasticity and/or a diastolic dysfunction.

In **a fourth aspect,** the invention pertains to a pharmaceutical composition comprising a therapeutically effective amount of a nucleic acid based miR-92 antagonist, or a pharmaceutically acceptable salt thereof, and a pharmaceutically acceptable carrier or diluent.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a micro-RNA (miR)-92 antagonist for use in the treatment or prevention of a heart disease in a subject, wherein the heart disease is caused by or associated with or caused by a reduced ventricular elasticity and/or a diastolic dysfunction.

In **a second aspect,** the invention pertains to a method of treating heart disease in a subject, the method comprising a step of administering a therapeutically effective amount of a micro-RNA (miR)-92 antagonist to the subject to treat the heart disease, and wherein the heart disease is caused by or associated with or caused by a reduced ventricular elasticity and/or a diastolic dysfunction.

In **a third aspect,** the invention pertains to the use of a micro-RNA (miR)-92 antagonist in the manufacture of a medicament for use in the treatment or prevention of a heart disease in a subject, wherein the heart disease is caused by or associated with or caused by a reduced ventricular elasticity and/or a diastolic dysfunction.

In an embodiment of the invention a heart disease is preferably heart failure. The term "heart failure" (abbreviated "HF") is well known by the skilled person. As used herein, the term relates preferably to an impaired systolic and/or diastolic function of the heart being accompanied by overt signs of heart failure as known to the person skilled in the art. Heart failure may be referred to herein as acute or chronic heart failure. Heart failure according to the present invention includes overt and/or advanced heart failure. In overt heart failure, the subject shows symptoms of heart failure as known to the person skilled in the art. In an embodiment of present invention, the term "heart failure" refers to heart failure with with preserved left ventricular ejection fraction (HFpEF). Hence, in a preferred embodiment, the invention provides embodiments wherein the heart disease is Heart Failure with preserved Ejection Fraction (HFpEF).

As used herein, the term "heart failure with preserved ejection fraction" or the abbreviation "HFpEF" has its general meaning in the art and refers to a complex syndrome characterized by heart failure (HF) signs and symptoms and a normal or near-normal left ventricular ejection fraction (FVEF). More specific diagnostic criteria include signs/symptoms of HF, objective evidence of diastolic dysfunction, disturbed left ventricular (FV) filling, structural heart disease, and elevated brain natriuretic peptides. Additional cardiac abnormalities can include subtle alterations of systolic function, impaired atrial function, chronotropic incompetence, or haemodynamic alterations, such as elevated pre-load volumes.

In some embodiments of the invention, the HFpEF is irrespective of the presence or absence of coronary artery disease in the subject to be treated. The term "coronary artery disease" refers to any disease of arteries that supply blood to the heart and/or arteries that surround the heart. For example, the term "coronary artery disease" includes thrombosis or a blood clot in the coronary arteries.

Furthermore, in additional or alternative embodiments of the invention, the heart disease is a non-ischemic heart disease and/or wherein the heart disease is not heart failure with reduced ejection fraction (HFrEF). Alternatively, it may be preferably, that the subject suffering from heart disease, which is to be treated, or prevented, is a subject having a non-ischemic etiology of heart disease. The term "non-ischemic" shall refer to an etiology of heart disease wherein the heart does not have an insufficient blood supply to one or more regions of the myocardium. It is preferred in context of the invention, that the heart disease is Heart Failure with preserved Ejection Fraction (HFpEF), and most preferably, wherein the heart failure is non-ischemic, therefore may be a non-ischemic HFpEF.

The terms "patient" and "subject" are used synonymously in the present disclosure. Provided herein are methods of treating in particular heart heart disease in a subject. As used herein, the term "subject" refers to any vertebrate including, without limitation, humans and other primates (e.g., chimpanzees and other apes and monkey species), farm animals (e.g. , cattle, sheep, pigs, goats and horses), domestic mammals (e.g. , dogs and cats), laboratory animals (e.g. , rodents such as mice, rats, and guinea pigs), and birds (e.g., domestic, wild and game birds such as chickens, turkeys and other gallinaceous birds, ducks, geese, and the like). In some embodiments, the subject is a mammal. In other embodiments, which are preferred, the subject is a human. In particular embodiments of the invention, the subject or patient to be treated is a patient not suffering from an ischemic heart disease.

A subject that can be treated according to the invention is preferably a subject characterized by having one or more of the following risk factors: sedentary lifestyle, obesity, high blood pressure, and/or diabetes.

In particular preferred embodiments of the invention, the subject treated according to the invention shows, and is distinguishable by, an abnormal measured exercise pulmonary artery pressure, such as an abnormal measured exercise pulmonary artery pressure of more than 30 mmHg.

In further particular preferred embodiments of the invention, the subject treated according to the invention shows, and is distinguishable by, an abnormal measured exercise pulmonary capillary wedge pressure, such as an abnormal measured exercise pulmonary capillary wedge pressure of at least 25 mmHg.

In yet another particular preferred embodiment of the invention, the subject treated according to the invention shows, and is distinguishable by, a normal measured resting pulmonary artery pressure or a normal measured resting pulmonary capillary wedge pressure.

In yet another particular preferred embodiment of the invention, the subject treated according to the invention shows, and is distinguishable by, symptoms of left ventricular diastolic dysfunction.

In yet another particular preferred embodiment of the invention, the subject treated according to the invention shows, and is distinguishable by, that the subject does not show symptoms of renal disease and/or cardiovascular disease.

The present invention relates to methods, compounds and compositions for the treatment of heart diseases as described and defined herein in subjects that are in need of such treatment. As used herein, the term "treatment" or "treat" refer to both prophylactic or preventive treatment as well as curative or disease modifying treatment, including treatment of patients at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular interval, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

A treatment or prevention in accordance with the present invention shall be understood to comprises the administration of a therapeutically effective amount of the miR-92 antagonist to the subject, and thereby treating or preventing the heart disease in the subject.

The term "miR-92" as used herein includes pri-miR-92, pre-miR-92, miR-92, miR-92a, miR-92b, miR-92a-3p, and hsa-miR-92a-3p.

Preferred in the context of the invention are such antagonists of miR-92, which are nucleic acid comprising compounds or compositions. Preferably such nucleic acids have the ability to bind to varying degrees to the miR-92 and thereby inhibit its biological function.

Oligonucleotide Inhibitors which are nucleic acid based antagonists of miR-92 are described in the following.

In the context of the present invention, the term "antagonists of miR-92" may refer preferably to an "oligonucleotide inhibitor", "antimir", "antagonist", "antisense oligonucleotide or ASO", "oligomer", "anti-microRNA oligonucleotide or AMO", or "mixmer" and such term is used broadly and encompasses an oligomer comprising ribonucleotides, deoxyribonucleotides, modified ribonucleotides, modified deoxyribonucleotides or a combination thereof, that inhibits the activity or function of the target miR-92 by fully or partially hybridizing to the miR-92, thereby repressing the function or activity of the target miR-92.

The activity of the oligonucleotide in modulating the function and/or activity of miR- 92 may be determined in vitro, ex vivo and/or in vivo. For example, when inhibition of miR-92 activity is determined in vitro, the activity may be determined using a dual luciferase assay. The dual luciferase assay can be any dual luciferase assay known in the art. The dual luciferase assay can be a commercially available dual luciferase assay. The dual luciferase assay, as exemplified by the commercially available product PsiCHECK^{™} (Promega), can involve placement of the miR recognition site in the 3' UTR of a gene for a detectable protein (e.g., renilla luciferase). The construct can be co-expressed with miR-92, such that inhibitor activity can be determined by change in signal. A second gene encoding a detectable protein (e.g., firefly luciferase) can be included on the same plasrmd, and the ratio of signals determined as an indication of the a timiR- 92 activity of a candidate oligonucleotide. In some embodiments, the oligonucleotide significantly inhibits such activity, as determined in the dual luciferase activity, at a concentration of about 50 nM or less, or in other embodiments, 40 nM or less, 20 nM or less, or 10 tiM or less. For example, the oligonucleotide may have an IC₅₀ for inhibition of miR-92 activity of about 50 nM or less, 40 nM or less, 30 nM or less, or 20 nM or less, as determined in the dual luciferase assay.

Alternatively, or in addition, the in vivo efficacy of the oligonucleotide inhibitor of miR-92 may also be determined in a suitable animal model. The animal model can be a rodent model (e.g., mouse or rat model). The oligonucleotide may exhibit at least 50% miR-92 target derepression at a dose of 50 mg/kg or less, 25 mg/kg or less, 10 mg/kg or less or 5 mg/kg or less. In such embodiments, the oligonucleotide may be dosed, delivered or administered to the non-human animal intravenously or subcutaneously or delivered locally such as local injection, and the oligonucleotide may be formulated in saline. The oligonucleotide inhibitor of miR-92 as provided herein can have increased in vivo efficacy in a particular tissue as compared to other oligonucleotide inhibitors of miR-92.

In these or other embodiments, the oligonucleotides of the present invention can be stable after administration, being detectable in the circulation and/or target organ for at least three weeks, at least four weeks, at least five weeks, or at least six weeks, or more, following administration. Thus, the oligonucleotide inhibitors of miR-92 provided herein may provide for less frequent administration, lower doses, and/or longer duration of therapeutic effect as compared to other oligonucleotide inhibitors of miR-92.

The nucleotide sequence of the oligonucleotide can be substantially complementary to a nucleotide sequence of an RNA, such as a mRNA or miRNA. The nucleotide sequence of the oligonucleotide can be fully complementary to a nucleotide sequence of an RNA, such as a mRNA or miRNA. In some embodiments, the miRNA is miR-92 or miR-92a. The oligonucleotide comprises at least one LNA, such as at least two, at least three, at least five, at least seven or at least nine LNAs. In some embodiments, the oligonucleotide comprises a mix of LNA and non- locked nucleotides. For example, the oligonucleotide may contain at least five or at least seven or at least nine locked nucleotides, and at least one non-locked nucleotide.

Generally, the length of the oligonucleotide and number and position of locked nucleotides can be such that the oligonucleotide reduces miR-92 function and/or activity, in some embodiments, the length of the oligonucleotide and number and position of locked nucleotides is such that the oligonucleotide reduces miR-92 function and/or activity at an oligonucleotide concentration of about 50 nM or less in the in vitro luciferase assay, or at a dose of about 50 mg/kg or less, or about 25 mg/kg or less in a suitable mouse or rat model, each as described. In some embodiments, the length of the oligonucleotide and number and position of locked nucleotides is such that the oligonucleotide reduces miR-92 activity as determined by target de-repression, at a dose of about 50 mg/kg or less, or about 25 mg/kg or less in a suitable mouse or rat model, such as described herein.

The oligonucleotide of the present invention can comprise a sequence of nucleotides in which the sequence comprises at least five LNAs, a LNA at the 5' end of the sequence, a LNA at the 3' end of the sequence, or any combination thereof. In one embodiment, the oligonucleotide comprises a sequence of nucleotides in which the sequence comprises at least five LNAs, a LNA at the 5' end of the sequence, a LNA at the 3' end of the sequence, or any combination thereof, wherein three or fewer of the nucleotides are contiguous LNAs. For example, the oligonucleotide comprises no more than three contiguous LN As. For example, the oligonucleotide may comprise a sequence with at least five LNAs, a LNA at the 5! end, a LNA at the 3' end, and no more than three contiguous LNAs. The oligonucleotide may comprise a sequence with at least five LN As, a LNA at the 5' end, a LNA at the 3' end, and no more than three contiguous LNAs, wherein the sequence is at least 16 nucleotides in length. The sequence can be substantially or completely complementary to a RNA, such as mRNA, or miRNA, wherein a substantially complementary sequence may have from 1 to 4 mismatches (e.g., 1 or 2 mismatches) with respect to its target sequence. In one embodiment, the target sequence is a miRNA, such that the oligonucleotide is a miRNA inhibitor, or antimiR. In one embodiment, the target sequence is a miR-92 sequence as provided herein.

In yet another embodiment, the oligonucleotide of the present invention can comprise a sequence complementary to the seed region of miR-92, wherein the sequence comprises at least five LNAs. The "seed region of a miRNA" is the portion spanning bases 2 to 9 at the 5' end of the miRNA. The oligonucleotide comprising a sequence complementary to the seed region of a miR-92, wherein the sequence comprises at least five LNAs, may comprise a LNA at the 5' end or a LNA at the 3' end, or both a LNA at the 5' end and 3' end. In one embodiment, the oligonucleotide comprising at least 5 LNAs, a LNA at the 5' end and/or a LNA at the 3' end, also has three or fewer consecutive LNAs. In some embodiments, the sequence is at least 16 nucleotides in length. The sequence complementary to the seed region of a miRNA can be substantially complementary or completely complementary.

The oligonucleotides of the present invention may comprise one or more locked nucleic acid (LNAs) residues, or "locked nucleotides," The oligonucleotide of the present invention can contain one or more locked nucleic acid (LNAs) residues, or "locked nucleotides," The oligonucleotides of the present invention may comprise one or more nucleotides containing other sugar or base modifications. The terms "locked nucleotide," "locked nucleic acid unit," "locked nucleic acid residue," "LNA" or "LNA unit" may be used interchangeably throughout the disclosure and refer to a bicyclic nucleoside analogue. For instance, suitable oligonucleotide inhibitors can be comprised of one or more "conformationally constrained" or bicyclic sugar nucleoside modifications (BSN) that confer enhanced thermal stability to complexes formed between the oligonucleotide containing BSN and their complementary target strand. LNAs are modified nucleotides or ribonucleotides that contain an extra bridge between the 2' and 4' carbons of the ribose sugar moiety resulting in a "locked" conformation, and/or bicyclic structure. In one embodiment, the oligonucleotide contains one or more LNAs having the structure shown by structure A below. Alternatively, or in addition, the oligonucleotide may contain one or more LNAs having the structure shown by structure B below. Alternatively, or in addition, the oligonucleotide contains one or more LNAs having the structure shown by structure C below.

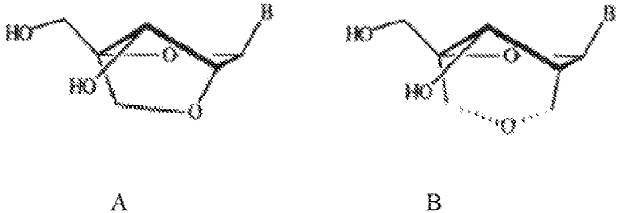

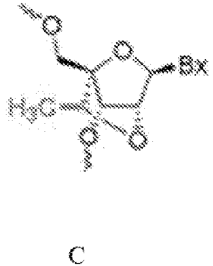

When referring to substituting a DNA or RNA nucleotide by its corresponding locked nucleotide in the context of the present invention, the term "corresponding locked nucleotide" is intended to mean that the DNA/RNA nucleotide has been replaced by a locked nucleotide containing the same naturally-occurring nitrogenous base as the DNA/RNA nucleotide that it has replaced or the same nitrogenous base that is chemically modified. For example, the corresponding locked nucleotide of a DNA nucleotide containing the nitrogenous base C may contain the same nitrogenous base C or the same nitrogenous base C that is chemically modified, such as 5-methylcytosine.

The term "non-locked nucleotide" refers to a nucleotide different from a locked-nucleotide, i.e. the term "non-locked nucleotide" includes a DNA nucleotide, an RNA nucleotide as well as a modified nucleotide where a base and/or sugar is modified except that the modification is not a locked modification.

Other suitable locked nucleotides that can be incorporated in the oligonucleotide inhibitors of miR-92 of the present invention include those described in U.S. Patent Nos. 6,403,566 and 6,833,361 , both of which are hereby incorporated by reference in their entireties.

In exemplary embodiments, the locked nucleotides have a 2' to 4' methylene bridge, as shown in structure A, for example. In other embodiments, the bridge comprises a methylene or ethylene group, which may be substituted, and which may or may not have an ether linkage at the 2' position.

The oligonucleotide inhibitors of miR-92 of the present invention may include modified nucleotides that have a base modification or substitution. The natural or unmodified bases in RNA are the purine bases adenine (A) and guanine (G), and the pyrimidine bases cytosine (C) and uracil (U) (DNA has thymine (T)). Modified bases, also referred to as heterocyclic base moieties, include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2 -propyl and other alkyl derivatives of adenine and guanine, 2-thiouracil, 2-thiothymine and 2-thioeytosine, 5-halouracil and cytosine, 5-propynyl uracil and cytosine and other alkynyl derivatives of pyrimidme bases, 6-azo uracil, cytosine and thymine, 5-uracil (pseudouracil), 4-thiouracil, 8-halo, 8-amino, 8-thiol, 8-thioalkyl, 8-hydroxyl and other 8- substituted adenines and guanines, 5-halo (including 5-bromo, 5-trifluoromethyl and other 5-substituted uracils and cytosines), 7-methylguanme and 7-methyladenine, 2-F-adenine, 2-aminoadenine, 8-azaguanine and 8-azaadenine, 7-deazaguanine and 7-deazaadenine and 3-deazaguanine and 3-deazaadenine. In certain embodiments, oligonucleotide inhibitors targeting miR-92 comprise one or more BSN modifications (i.e., LNAs) in combination with a base modification (e.g. 5-methyl cytidine).

The oligonucleotide inhibitors of miR-92 of the present invention may include nucleotides with modified sugar moieties. Representative modified sugars include carbocyclic or acyclic sugars, sugars having substituent groups at one or more of their 2', 3' or 4' positions and sugars having substituents in place of one or more hydrogen atoms of the sugar. In certain embodiments, the sugar is modified by having a substituent group at the 2' position. In additional embodiments, the sugar is modified by having a substituent group at the 3' position. In other embodiments, the sugar is modified by having a substituent group at the 4' position. It is also contemplated that a sugar may have a modification at more than one of those positions, or that an oligonucleotide inhibitor may have one or more nucleotides with a sugar modification at one position and also one or more nucleotides with a sugar modification at a different position.

The oligonucleotide may comprise, consist essentially of, or consist of, an antisense sequence to miR-92. In one embodiment, the oligonucleotide comprises an antisense sequence directed to miR-92. For example, the oligonucleotide can comprise a sequence that is at least partially complementary to a mature miR-92 sequence, e.g. at least about 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, or 99% complementary to a mature miR-92 sequence. In one embodiment, the oligonucleotide inhibitor as provided herein comprises a sequence that is at least 75% complementary to a mature miR-92 sequence. In one embodiment, the oligonucleotide inhibitor as provided herein comprises a sequence that is at least 85% complementary to a mature miR-92 sequence. In one embodiment, the oligonucleotide inhibitor as provided herein comprises a sequence that is at least 95% complementary to a mature miR-92 sequence. In one embodiment, the oligonucleotide inhibitor as provided herein comprises a sequence that is at least 75% complementary to a miR-92 inhibitor selected from those listed in Table 2 of WO/2018/183127 (Table 2 of WO/2018/183127 [filed as PCT/US2018/024167] is incorporated herein by reference). In one embodiment, the oligonucleotide inhibitor as provided herein comprises a sequence that is at least 85% complementary to a miR-92 inhibitor selected from those listed in Table 2 of WO/2018/183127. In one embodiment, the oligonucleotide inhibitor as provided herein comprises a sequence that is at least 95% complementary to a miR-92 inhibitor selected from those listed in Table 2 of WO/2018/183127. In one embodiment, the oligonucleotide inhibitor as provided herein comprises a sequence that is 100% or fully complementary to a mature miR-92 sequence. It is understood that the sequence of the oligonucleotide inhibitor is considered to be complementary to miR-92 even if the oligonucleotide inhibitor sequence includes a modified nucleotide instead of a naturally-occurring nucleotide. For example, if a mature sequence of miR- 92 comprises a guanosine nucleotide at a specific position, the oligonucleotide inhibitor may comprise a modified cytidme nucleotide, such as a locked cytidine nucleotide or 2'-fluoro- cytidine, at the corresponding position. For the purposes of the invention any description of compounds or compositions for inhibiting miR-92 as described in WO/2018/183127 is hereby incorporated by reference to this patent publication, and in particular incorporated are paragraphs 12, and paragraphs including 38 to and including 84 (including table 2 therein).

In one embodiment, the oligonucleotide inhibitor as provided herein comprises a sequence that is at least 75% complementary to a mature miR-92 sequence of SEQ ID NO: 1. In one embodiment, the oligonucleotide inhibitor as provided herein comprises a sequence that is at least 85% complementary to a mature miR-92 sequence of SEQ ID NO: 1. In one embodiment, the oligonucleotide inhibitor as provided herein comprises a sequence that is at least 95% complementary to a mature miR-92 sequence of SEQ ID NO: 1, and a preferred embodiment pertains to a miR-92 inhibitor comprising, or consisting (essentially) of, the sequence shown in SEQ ID NO: 1.

In certain embodiments, the oligonucleotide comprises a nucleotide sequence that is completely complementary to a nucleotide sequence of miR-92. In particular embodiments, the oligonucleotide comprises, consists essentially of, or consists of the nucleotide sequence complementary to miR-92. In this context, "consists essentially of includes the optional addition of nucleotides (e.g., one or two) on either or both of the 5' and 3' ends, so long as the additional nucleotide(s) do not substantially affect (as defined by an increase in IC₅₀ of no more than 20%) the oligonucleotide's inhibition of the target miRNA activity in the dual luciferase assay or animal (e.g., mouse) model.

The oligonucleotide can generally have a nucleotide sequence designed to target mature miR-92. The oligonucleotide may, in these or other embodiments, also or alternatively be designed to target the pre- or pri-miRNA forms of miR-92. In certain embodiments, the oligonucleotide may be designed to have a sequence containing from 1 to 5 (e.g., 1, 2, 3, or 4) mismatches relative to the fully complementary (mature) miR-92 sequence. In certain embodiments, such antisense sequences may be incorporated into shRNAs or other RNA structures containing stem and loop portions, for example. [0057] The oligonucleotide can be from 8 to 20 nucleotides in length, from 15 to 50 nucleotides in length, from 18 to 50 nucleotides in length, from 10 to 18 nucleotides in length, or from 11 to 16 nucleotides in length. The oligonucleotide in some embodiments is about 8, about 9, about 10, about 11, about 12, about 13, about 14, about 15, about 16, about 17, or about 18 nucleotides in length, in one embodiment, the present invention provides an oligonucleotide inhibitor of miR- 92 that has a length of 11 to 16 nucleotides. In various embodiments, the oligonucleotide inhibitor targeting miR-92 is 11,12,13,14,15, or 16 nucleotides in length, in one embodiment, the oligonucleotide inhibitor of miR-92 has a length of 12 nucleotides, in some embodiments, the oligonucleotide inhibitor of miR-92 is at least 16 nucleotides in length.

Generally, the number and position of LNA can be such that the oligonucleotide reduces miR-92 activity or function. In one embodiment, the number and position of LNAs is such that the oligonucleotide has an increased efficacy relative to a control. In some embodiments, efficacy is a capacity for producing a beneficial or desired result (e.g., clinical result). The beneficial or desired result can be a reduction, amelioration, or removal of a symptom or symptoms of a disease or condition. The beneficial or desired result can be a inhibition, reduction, amelioration, or removal of the activity or function of miR-92. The increased efficacy can be increased in vivo, in vitro, or ex vivo. The control can be an oligonucleotide containing the same sequence as the oligonucleotide comprising LNAs as provided herein but no chemical modifications. The control can be an oligonucleotide containing the same sequence as the oligonucleotide comprising LNAs as provided herein but a different chemical modification motif or pattern. The control can be an oligonucleotide containing the same sequence as the oligonucleotide comprising LNAs as provided herein but a different number and/or position of LNAs. The control can be an oligonucleotide containing the same sequence as well as number and/or position of LNAs, but a different additional modification such as the presence of one or more 5-methylcytosines.

The oligonucleotide inhibitors of miR-92 as provided herein generally contain at least about 2, at least about 3, at least about 4, at least about 5, at least about 7, or at least about 9 LNAs, but in various embodiments is not fully comprised of LN As. Generally, the number and position of LNAs is such that the oligonucleotide reduces mRNA or miRNA function or activity. In certain embodiments, the oligonucleotide does not contain a stretch of nucleotides with more than four, or more than three, contiguous LNAs. For example, the oligonucleotide comprises no more than three contiguous LNAs. In these or other embodiments, the oligonucleotide can comprise a region or sequence that is substantially or completely complementary to a miRNA seed region, in which the region or sequence comprises at least two, at least three, at least four, or at least five locked nucleotides.

In certain embodiments, the oligonucleotide inhibitor of miR-92 contains at least 1, at least 2, at least 3, at least 4, or at least 5 DNA nucleotides. In one embodiment, the oligonucleotide inhibitor comprises at least one I.NA, wherein each non-locked nucleotide in the oligonucleotide inhibitor is a DNA nucleotide. In one embodiment, the oligonucleotide inhibitor comprises at least two LNAs, wherein each non-locked nucleotide in the oligonucleotide inhibitor is a DNA nucleotide. In one embodiment, at least the second nucleotide from the 5' end of the oligonucleotide inhibitor is a DNA nucleotide. In one embodiment, at least 1, at least 2, at least 3, at least 4, or at least 5 DNA nucleotides in an oligonucleotide as provided herein contains a nitrogenous base that is chemically modified. In one embodiment, the second nucleotide from the 5' end of an oligonucleotide inhibitor as provided herein contains a nitrogenous base that is chemically modified. The chemically modified nitrogenous base can be 5-methylcytosine. In one embodiment, the second nucleotide from the 5' end is a 5-methylcytosine. In one embodiment, an oligonucleotide inhibitor as provided herein comprises a 5-methylcytosine at each LNA that is a cytosine.

In one embodiment, an oligonucleotide inhibitor of miR-92 as provided herein comprises a sequence of 12 to 16 nucleotides, wherein the sequence is at least partially or fully complementary to a mature sequence of miR-92, in which from the 5' end to the 3' end of the oligonucleotide, at least the first and last nucleotide positions are LNAs. In certain embodiments, the oligonucleotide inhibitor of miR-92 has a length of 12 nucleotides. In certain embodiments, the oligonucleotide inhibitor of miR-92 has a length of 13 nucleotides. In certain embodiments, the oligonucleotide inhibitor of miR-92 has a length of 14 nucleotides. In certain embodiments, the oligonucleotide inhibitor of miR-92 has a length of 15 nucleotides. In certain embodiments, the oligonucleotide inhibitor of miR-92 has a length of 16 nucleotides. The oligonucleotide can have a full or partial (i.e., one or more) phosphorothioate backbone. The oligonucleotide can further comprise any additional modification as provided herein including but not limited to one or more chemically modified nitrogenous bases, a 5' and/or 3' cap structure, a pendent lipophilic group and/or 2' deoxy, 2' O-alkyl or 2' halo modification(s). In certain embodiments, the oligonucleotide inhibitor of miR-92 comprising a sequence of from 12 to 16 nucleotides comprises at least one nucleotide with a chemically modified nitrogenous base. The chemically modified nitrogenous base can be a methylated base. In certain embodiments, the chemically modified nitrogenous base is 5-methyIcytosine. In one embodiment, each LNA that is a cytosme is a 5- methylcytosine. In certain embodiments, an oligonucleotide inhibitor as provided herein comprising at least one nucleotide with a chemically modified nitrogenous base (e.g., 5-methylcytosine) shows increased efficacy as compared to the same oligonucleotide inhibitor lacking the chemically modified nitrogenous base. The increased efficacy can be an increased reduction or inhibition of miR-92 function and/or activity. The increased efficacy can be in vivo, ex vivo and/or in vitro.

In one embodiment, the oligonucleotide can comprise a sequence of 13 to 16 nucleotides, in which from the 5' end to the 3' end of the oligonucleotide, positions 1, 6, 10, 11 and 13 are LNAs, and the remaining positions are non-locked nucleotides, wherein the oligonucleotide is at least partially complementary to a miRNA or a seed region of a miRNA, in which the miRNA may in some embodiments, be miR-92. The oligonucleotide can be fully complementar to the miRN A, in which the miRN A may in some embodiments, be miR-92. In some embodiments, at least one non-locked nucleotide comprises a nitrogenous base that is chemically modified. In certain embodiments, the oligonucleotide inhibitor comprises a nucleotide containing a chemically modified nitrogenous base at a second nucleotide position from the 5' end to the 3' end of the oligonucleotide. In certain embodiments, the second nucleotide position is a cytosme and the chemically modified nitrogenous base is a 5-methylcytosine. In one embodiment, the presence of the chemically modified nitrogenous base(s) (e.g., 5-methylcytosine) in the oligonucleotide inhibitor has increased in vivo or in vitro efficacy as compared to an oligonucleotide with the same number and/or position of LNAs but no chemically modified nitrogenous base (e.g., 5-methylcytosine). The increased efficacy can be an increased reduction of miR-92 function and/or activity.

In another embodiment, the oligonucleotide can comprise at least 16 nucleotides, in which from the 5' end to the 3' end of the oligonucleotide, positions 1, 3, 6, 8, 10, 11, 13, 14, and 16 are LNAs, and the remaining positions are non-locked nucleotides, the oligonucleotide is at least partially complementary to a miRNA or a seed region of a miRNA, in which the miRNA may in some embodiments, be miR-92. The oligonucleotide can be fully complementary to the miRNA, in which the miRNA may in some embodiments, be miR-92. In some embodiments, the second nucleotide from the 5' end comprises a nitrogenous base that is chemically modified (e.g. 5-methylcytosine). In one embodiment, the presence of the chemically modified nitrogenous base(s) (e.g., 5-methylcytosine) in the oligonucleotide inhibitor has increased in vivo or in vitro efficacy as compared to an oligonucleotide with the same number and/or position of LNAs but no chemically modified nitrogenous base (e.g., 5-methylcytosine). The increased efficacy can be an increased reduction of miR-92 function and/or activity.

In another embodiment, the oligonucleotide can comprise at least 16 nucleotides, in which from the 5' end to the 3' end of the oligonucleotide, positions 1, 5, 6, 8, 10, 11, 13, 15, and 16 are LNAs, and the remaining positions are non-locked nucleotides, the oligonucleotide is at least partially complementary to a miRNA or a seed region of a miRNA, in which the miRNA may in some embodiments, be miR-92. The oligonucleotide can be fully complementary to the miRNA, m which the miRNA may in some embodiments, be miR-92. In some embodiments, the second nucleotide from the 5' end comprises a nitrogenous base that is chemically modified (e.g. 5-methylcytosine). In one embodiment, the presence of the chemically modified nitrogenous base(s) (e.g., 5-methylcytosine) in the oligonucleotide inhibitor has increased in vivo or in vitro efficacy as compared to an oligonucleotide with the same number and/or position of LNAs but no chemically modified nitrogenous base (e.g., 5-methylcytosine). The increased efficacy can be an increased reduction of miR-92 function and/or activity.

In another embodiment, the oligonucleotide can comprise at least 16 nucleotides, in which from the 5' end to the 3! end of the oligonucleotide, positions 1, 3, 6, 9, 10, 11, 13, 14, and 16 are LNAs, and the remaining positions are non-locked nucleotides, the oligonucleotide is at least partially complementary to a m iRNA or a seed region of a m iRNA, in which the miRN A may in some embodiments, be miR-92. The oligonucleotide can be fully complementary to the miRNA, in which the miRNA may in some embodiments, be miR-92. In some embodiments, the second nucleotide from the 5' end comprises a nitrogenous base that is chemically modified (e.g. 5-methylcytosine). In one embodiment, the presence of the chemically modified nitrogenous base(s) (e.g., 5-methylcytosine) in the oligonucleotide inhibitor has increased in vivo or in vitro efficacy as compared to an oligonucleotide with the same number and/or position of LNAs but no chemically modified nitrogenous base (e.g., 5-methylcytosine). The increased efficacy can be an increased reduction of miR-92 function and/or activity. [0066] In some embodiments, an oligonucleotide inhibitor of miR-92 shows at least about 0.5%, at least about 1%, at least about 2%, at least about 3%, at least about 4%, at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, or at least about 99% greater inhibition of the function and/or activity of the target miR-92 as compared to other inhibitors of the target miR-92. The improvement or increase can be in vitro, ex vivo and/or in vivo.

In some embodiments, an oligonucleotide inhibitors of miR-92 comprising a 5-methylcytosine produces at least about 0.5%, at least about 1%, at least about 2%, at least about 3%, at least about 4%, at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, at least about 90%, or at least about 99% of an increase or improvement in the reduction of function and/or activity of the target miR-92 as compared to an oligonucleotide with the same nucleotide sequence as well as LNA/DNA pattern but lacking a 5-methylcyotsine. The improvement or increase can be in vitro, ex vivo and/or in vivo. In some cases, all LNA cytosines in an oligonucleotide inhibitor as provided herein is a 5-methylcytosine LNA.

In some embodiments for non-locked nucleotides, the nucleotide may contain a 2' modification with respect to a 2' hydroxyl. For example, the 2' modification may be 2' deoxy. Incorporation of 2' -modified nucleotides in antisense oligonucleotides may increase resistance of the oligonucleotides to nucleases. Incorporation of 2'-modified nucleotides in antisense oligonucleotides may increase their thermal stability with complementary RNA. Incorporation of 2'-modified nucleotides in antisense oligonucleotides may increase both resistance of the oligonucleotides to nucleases and their thermal stability with complementary RNA. Various modifications at the 2' positions may be independently selected from those that provide increased nuclease sensitivity, without compromising molecular interactions with the RNA target or cellular machinery. Such modifications may be selected on the basis of their increased potency in vitro, ex vivo or in vivo. Exemplary methods for determining increased potency (e.g., IC₅₀) for miR-92 inhibition are described herein, including, but not limited to, the dual luciferase assay and in vivo miR-92 abundance or target de-repression. [0069] In some embodiments the 2' modification may be independently selected from O-alkyl (which may be substituted), halo, and deoxy (H). Substantially all, or all, nucleotide 2' positions of the non-locked nucleotides may be modified in certain embodiments, e.g. , as independently selected from O-alkyl (e.g., O-methyl), halo (e.g., fluoro), deoxy (H), and ammo. For example, the 2' modifications may each be independently selected from O-methyl (OMe) and fluoro (F). In exemplary embodiments, purine nucleotides each have a 2' OMe and pyrimidine nucleotides each have a 2'-F. In certain embodiments, from one to about five 2' positions, or from about one to about three 2' positions are left unmodified (e.g., as 2' hydroxyls).

2' modifications in accordance with the invention can also include small hydrocarbon substituents. The hydrocarbon substituents include alkyl, alkenyl, alkynyl, and alkoxyalkvl, where the alkyl (including the alkyl portion of alkoxy), alkenyl and alkynyl may be substituted or unsubstituted. The alkyl, alkenyl, and alkynyl may be CI to CIO alkyl, alkenyl or alkynyl, such as CI, C2, or C₃. The hydrocarbon substituents may mclude one or two or three non-carbon atoms, which may be independently selected from nitrogen (N), oxygen (O), and/or sulfur (S). The 2' modifications may further include the alkyl, alkenyl, and alkynyl as O-alkyl, O-alkenyl, and O-alkynyl.

Exemplary 2' modifications in accordance with the invention can include 2'-o-alkyl (CI - 3 alkyl, such as 2' OMe or 2'OEt), 2'-o-methoxyethyl (2!-o-MOE), 2'-o-aminopropyl (2'-o-AP), 2!-o-dimethylaminoethyl (2'-Q-DMAOE), 2!-o-dimethyiaminopropyi (2'-o-DMAP), 2!-o-dimethyiaminoethyloxyethyl (2!-o-DMAEOE), or 2!-o-N-methylacetamido (2'-o-NMA) substitutions.

In certain embodiments, the oligonucleotide contains at least one 2' -halo modification (e.g., in place of a 2' hydroxyl), such as 2' -fluoro, 2'-chloro, 2'-bromo, and 2'-iodo. In some embodiments, the 2' halo modification is fluoro. The oligonucleotide may contain from 1 to about 5 2' -halo modifications (e.g., fluoro), or from 1 to about 3 2'-halo modifications (e.g., fluoro). In some embodiments, the oligonucleotide contains all 2'-fluoro nucleotides at non-locked positions, or 2'-fluoro on all non-locked pyrimidine nucleotides. In certain embodiments, the 2' -fluoro groups are independently di-, tri-, or un-methylated.

The oligonucleotide may have one or more 2' -deoxy modifications (e.g., H for 2' hydroxy!), and in some embodiments, contains from 2 to about 10 2'-deoxy modifications at non- locked positions, or contains 2' deoxy at all non-locked positions.

In exemplary embodiments, the oligonucleotide contains 2' positions modified as 2'OMe in non-locked positions. Alternatively, non-locked purine nucleotides can be modified at the 2' position as 2'OMe, with non-locked pyrimidine nucleotides modified at the 2' position as 2'-fluoro.

in certain embodiments, the oligonucleotide further comprises at least one terminal modification or "cap." The cap may be a 5' and/or a 3'-cap structure. The terms "cap" or "end-cap" include chemical modifications at either terminus of the oligonucleotide (with respect to terminal ribonucleotides), and includes modifications at the linkage between the last two nucleotides on the 5' end and the last two nucleotides on the 3' end. The cap structure as described herein may increase resistance of the oligonucleotide to exonucleases without compromising molecular interactions with the target miR-92 or cellular machinery. Such modifications may be selected on the basis of their increased potency in vitro or in vivo. The cap can be present at the 5'-terminus (5'-cap) or at the 3 '-terminus (3'-cap) or can be present on both ends. In certain embodiments, the 5'- and/or 3 '-cap is independently selected from phosphorothioate monophosphate, abasic residue (moiety), phosphorothioate linkage, 4'-thio nucleotide, carbocyclic nucleotide, phosphorodithioate linkage, inverted nucleotide or inverted abasic moiety (2'-3' or 3'- 3'), phosphorodithioate monophosphate, and methylphosphonate moiety. The phosphorothioate or phosphorodithioate Imkage(s), when part of a cap structure, are generally positioned between the two terminal nucleotides on the 5' end and the two terminal nucleotides on the 3' end.

In certain embodiments, the oligonucleotide has at least one terminal phosphorothioate monophosphate. The phosphorothioate monophosphate may support a higher potency by inhibiting the action of exonucleases. The phosphorothioate monophosphate may be at the 5' and/or 3' end of the oligonucleotide. A phosphorothioate monophosphate is defined by the following structures, where B is base, and R is a 2' modification as described above:

Where the cap structure can support the chemistry of a locked nucleotide, the cap structure may incorporate a LNA as described herein.

Phosphorothioate linkages may be present in some embodiments, such as between the last two nucleotides on the 5' and the 3' end (e.g., as part of a cap structure), or as alternating with phosphodi ester bonds, in these or other embodiments, the oligonucleotide may contain at least one terminal abasic residue at either or both the 5' and 3' ends. An abasic moiety does not contain a commonly recognized purine or pyrimidine nucleotide base, such as adenosine, guanine, cytosme, uracil or thymine. Thus, such abasic moieties lack a nucleotide base or have other non- nucleotide base chemical groups at the Γ position. For example, the abasic nucleotide maybe a reverse abasic nucleotide, e.g., where a reverse abasic phosphoramidite is coupled via a 5' amidite (instead of 3' amidite) resulting in a 5 '-5' phosphate bond. The structure of a reverse abasic nucleoside for the 5' and the 3' end of a polynucleotide is shown below.

The oligonucleotide may contain one or more phosphorothioate linkages, Phosphorothioate linkages can be used to render oligonucleotides more resistant to nuclease cleavage. For example, the polynucleotide may be partially phosphorothioate-linked, for example, phosphorothioate linkages may alternate with phosphodiester linkages. In certain embodiments, however, the oligonucleotide is fully phosphorothioate-linked. In other embodiments, the oligonucleotide has from one to five or one to three phosphate linkages.

In one embodiment, the oligonucleotide inhibitor of the present invention comprises, or consists essentially of, a sequence selected from Table 2 in WO 2018/183127 which table is incorporated herein by reference in its entirety. Such oligonucleotide preferably comprises at least one non-locked nucleotide that is 2' O-alkyl or 2' halo modified. In some embodiments, the oligonucleotide comprises at least one LNA that has a 2' to 4' methylene bridge. In some embodiments, the oligonucleotide has a 5' cap structure, 3' cap structure, or 5' and 3 ' cap structure. In yet other embodiments, the oligonucleotide comprises a pendent lipophilic group.

The oligonucleotide may be incorporated within a variety of macromolecular assemblies or compositions. Such complexes for delivery may include a variety of liposomes, nanoparticles, and micelles, formulated for delivery to a subject. The complexes may include one or more fusogenic or lipophilic molecules to initiate cellular membrane penetration. Such molecules are described, for example, in US Patent No. 7,404,969 and US Patent No. 7,202,227, which are hereby incorporated by reference in their entireties. Alternatively, the oligonucleotide may further comprise a pendant lipophilic group to aid cellular delivery, such as those described in WO 2010/129672, which is hereby incorporated by reference.

In **a fourth aspect,** the invention pertains to a pharmaceutical composition comprising a therapeutically effective amount of a nucleic acid based miR-92 antagonist, or a pharmaceutically acceptable salt thereof, and a pharmaceutically acceptable carrier or diluent.

Such pharmaceutical composition can be used in methods of treatment as described above for miR-92 antagonists in a corresponding manner. Hence, the pharmaceutical composition of the invention is for use in the treatment or prevention of a heart disease in a subject, wherein the heart disease is caused by or associated with a reduced ventricular elasticity and/or a diastolic dysfunction, as described herein above.

Preferably, in some embodiments, the pharmaceutically acceptable carrier comprises a colloidal dispersion system, macromolecular complex, nano capsule, microsphere, bead, oil-in-water emulsion, micelle, mixed micelle, or liposome.

n some embodiments, an "effective dose" or "therapeutically effective amount" is an amount sufficient to affect a beneficial or desired clinical result, e.g. treatment of heart failure. An "effective amount" can be an amount sufficient or required to substantially reduce, eliminate or ameliorate a symptom or symptoms of a disease and/or condition. This can be relative to an untreated subject. An "effective dose" can be an amount sufficient or required to slow, stabilize, prevent, or reduce the severity of the heart failure or symptoms of heart failure in a subject. This can be relative to an untreated subject. An effective dose of an oligonucleotide disclosed herein may be from about 0.001 mg/kg to about 100 mg/kg, about 0.01 mg/kg to about 10 mg/kg, about 0.1 mg/kg to about 10 mg/kg, about 1 mg/kg to about 10 mg/kg, about 2,5 mg/kg to about 50 mg/kg, or about 5 mg/kg to about 25 mg/kg. The precise determination of what would be considered an effective dose may be based on factors individual to each subject, including their size, age, and nature of the oligonucleotide {e.g. melting temperature, LNA content, etc.). Therefore, dosages can be readily ascertained by those of ordinary skill in the art from this disclosure and the knowledge in the art. In some embodiments, the methods comprise administering an effective dose of the pharmaceutical composition 1, 2, 3, 4, 5, or 6 times a day. In some embodiments, administration is 1, 2, 3, 4, 5, 6, or 7 times a week. In other embodiments, administration is biweekly or monthly.

Colloidal dispersion systems, such as macromolecule complexes, nanocapsules, microspheres, beads, and lipid-based systems including oil-in-water emulsions, micelles, mixed micelles, and liposomes, may be used as delivery vehicles for the oligonucleotide inhibitors of miR-92 function.

In certain embodiments, liposomes used for delivery are amphoteric liposomes such SMARTICLES^{®} (Marina Biotech, Inc.) which are described in detail in U.S. Pre-grant Publication No, 20110076322. The surface charge on the SMARTICLES^{®} is fully reversible which make them particularly suitable for the delivery of nucleic acids. SMARTICLES^{®} can be delivered via injection, remain stable, and aggregate free and cross cell membranes to deliver the nucleic acids.

Any of the miR-92 oligonucleotide inhibitors described herein can be delivered to a target cell (e.g., a heart cell, fibrocyte, fibroblast, keratinocyte or endothelial cell) by delivering to the cell an expression vector encoding the miR-92 oligonucleotide inhibitor. A "vector" is a composition of matter which can be used to deliver a nucleic acid of interest to the interior of a cell. Numerous vectors are known in the art including, but not limited to, linear polynucleotides, polynucleotides associated with ionic or amphiphilic compounds, plasmids, and viruses. The nucleic acid inhibitor expression sequence is operably linked to a promoter for its expression. In certain embodiments, the promoter operably linked to a polynucleotide encoding an oligonucleotide inhibitor described herein (e.g. , oligonucleotide inhibitors of miR-92a) can be an inducible promoter. Inducible promoters are known in the art and include, but are not limited to, tetracycline promoter, metal lothionein IIA promoter, heat shock promoter, steroid/thyroid hormone/retinoic acid response elements, the adenovirus late promoter, and the inducible mouse mammary tumor virus LTR. Methods of delivering expression constructs and nucleic acids to cells are known in the art.

One will generally desire to employ appropriate salts and buffers to render delivery vehicles stable and allow for uptake by target cells. Aqueous compositions of the present invention can comprise an effective amount of the delivery vehicle comprising the inhibitor polynucleotides (e.g. liposomes or other complexes or expression vectors) dissolved or dispersed in a pharmaceutically acceptable carrier or aqueous medium. The phrases "pharmaceutically acceptable" or "pharmacologically acceptable" refers to molecular entities and compositions that do not produce adverse, allergic, or other untoward reactions when administered to an animal or a human. As used herein, "pharmaceutically acceptable earner" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present invention, its use in therapeutic compositions is contemplated. Supplementary active ingredients also can be incorporated into the compositions, provided they do not inactivate the oligonucleotides of the compositions.

The oligonucleotide inhibitors of miR-92 of the present invention include classic pharmaceutical preparations. Administration of these compositions according to the present invention may be via any common route so long as the target tissue is available via that route. This includes oral, nasal, or buccal. Alternatively, administration may be by intradermal, subcutaneous, intramuscular, intraperitoneal, intraarterial, or intravenous injection. In some embodiments, the pharmaceutical composition is directed injected into lung or cardiac tissue. In any of the embodiments provided herein, administration of the oligonucleotide is performed by intravenous administration, subcutaneous administration, intracardiac administration, or intracoronary administration. In some embodiments, the intracoronary administration is retrograde. In some embodiments, the intracoronary administration is anterograde.

Pharmaceutical compositions comprising a miR-92 inhibitor may also be administered by catheter systems or systems that isolate coronary/pulmonary circulation for delivering therapeutic agents to the heart and lungs. Various catheter systems for delivering therapeutic agents to the heart and coronary vasculature are known in the art. Some non-limiting examples of catheter-based delivery methods or coronary isolation methods suitable for use in the present invention are disclosed in U. S. Patent No, 6,416,510; U.S. Patent No. 6,716,1 96; U.S. Patent No. 6,953,466, WO 2005/082440, WO 2006/089340, U.S. Patent Publication No, 2007/0203445, U.S. Patent Publication No. 2006/0148742, and U.S. Patent Publication No. 2007/0060907, which are all herein incorporated by reference i their entireties. Such compositions would normally be administered as pharmaceutically acceptable compositions as described herein.

The oligonucleotide inhibitors of miR-92 may also be administered parenterally or intraperitoneally. By way of illustration, solutions of the oligonucleotide inhibitors of miR-92 as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinaiy conditions of storage and use, these preparations generally contain a preservative to prevent the growth of microorganisms.

The pharmaceutical forms suitable for injectable use, catheter delivery, or inhalational delivery include, for example, sterile aqueous solutions or dispersions and sterile powders for the extemporaneous preparation of sterile injectable solutions or dispersions (e.g. aerosols, nebulizer solutions). Generally, these preparations are sterile and fluid to the extent that easy injectability or aerosolization/nebulization exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof!, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chJorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin,

Sterile injectable solutions may be prepared by incorporating the active compounds in an appropriate amount into a solvent along with any other ingredients (for example as enumerated above) as desired, followed by filtered sterilization. Generally, dispersions are prepared by incorporating the various sterilized active ingredients into a sterile vehicle which contains the basic dispersion medium and the desired other ingredients, e.g., as enumerated above. In the case of sterile powders for the preparation of sterile injectable solutions, the preferred methods of preparation include vacuum-drying and freeze-drying techniques which yield a powder of the active ingredient(s) plus any additional desired ingredient from a previously sterile-filtered solution thereof. In some embodiments, sterile powders can be administered directly to the subject (i.e. without reconstitution in a diluent), for example, through an insufflator or inhalation device.

The compositions of the present invention generally may be formulated in a neutral or salt form. Pharmaceuticaliy-acceptabie salts include, for example, acid addition salts (formed with the free amino groups of the protein) derived from inorganic acids (e.g., hydrochloric or phosphoric acids), or from organic acids (e.g., acetic, oxalic, tartaric, mandeiic, and the like). Salts formed with the free carboxyl groups of the protein can also be derived from inorganic bases (e.g., sodium, potassium, ammonium, calcium, or ferric hydroxides) or from organic bases (e.g., isopropylamine, trimethyiamme, histidme, procaine and the like).

Upon formulation, solutions are preferably administered in a manner compatible with the dosage formulation and in such amount as is therapeutically effective. The formulations may easily be administered in a variety of dosage forms such as injectable solutions, drug release capsules, unit dose inhalers, and the like. For parenteral administration in an aqueous solution, for example, the solution generally is suitably buffered and the liquid diluent first rendered isotonic for example with sufficient saline or glucose. Such aqueous solutions may be used, for example, for intravenous, intramuscular, subcutaneous, intraarterial, and intraperitoneal administration. Preferably, sterile aqueous media are employed as is known to those of skill in the art, particularly in light of the present disclosure. By way of illustration, a single dose may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicit, and general safety and purity standards as required by FDA Office of Biologies standards.

The composition or formulation may employ a plurality of therapeutic oligonucleotides, including at least one described herein. For example, the composition or formulation may employ at least 2, 3, 4, or 5 miR-92 inhibitors described herein. In another embodiment, an oligonucleotide of the present invention may be used in combination with other therapeutic modalities. Combinations may also be achieved by contacting a cell with more than one distinct composition or formulation, at the same time. Alternatively, combinations may be administered sequentially.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1: An miR-92 antagonist for use in the treatment or prevention of a heart disease in a subject, wherein the heart disease is caused by or associated with or caused by a reduced ventricular elasticity and/or a diastolic dysfunction.
Item 2:The miR-92 antagonist for use of item 1, wherein the heart disease is Heart Failure with preserved Ejection Fraction (HFpEF).
Item 3:The miR-92 antagonist for use of item 2, wherein the heart disease is HFpEF irrespective of the presence or absence of coronary artery disease
Item 4:The miR-92 antagonist for use of any one of items 1 to 3, wherein the heart disease is a non-ischemic heart disease and/or wherein the heart disease is not heart failure with reduced ejection fraction (HFrEF).
Item 5:The miR-92 antagonist for use of any one of items 1 to 4, wherein the heart disease is Heart Failure with preserved Ejection Fraction (HFpEF), preferably non-ischemic HFpEF. Item 6:The miR-92 antagonist for use of any one of items 1 to 5, wherein the subject does not suffer from an ischemic heart disease.
Item 7:The miR-92 antagonist for use of any one of items 1 to 6, wherein the subject is characterized by having one or more of the following risk factors: sedentary lifestyle, obesity, high blood pressure, and/or diabetes.
Item 8: The miR-92 antagonist for use of any one of items 1 to 7, wherein the subject shows an abnormal measured exercise pulmonary artery pressure, such as an abnormal measured exercise pulmonary artery pressure of more than 30 mmHg.
Item 9:The miR-92 antagonist for use of any one of items 1 to 8, wherein the subject shows an abnormal measured exercise pulmonary capillary wedge pressure, such as an abnormal measured exercise pulmonary capillary wedge pressure of at least 25 mmHg.
Item 10: The miR-92 antagonist for use of any one of items 1 to 9, wherein the subject shows a normal measured resting pulmonary artery pressure or a normal measured resting pulmonary capillary wedge pressure.
Item 11: The miR-92 antagonist for use of any one of items 1 to 10, wherein the subject shows symptoms of left ventricular diastolic dysfunction.
Item 12: The miR-92 antagonist for use of any one of items 1 to 11, wherein the subject does not show symptoms of renal disease or cardiovascular disease.
Item 13: The miR-92 antagonist for use of any one of items 1 to 12, wherein the treatment or prevention comprises the administration of a therapeutically effective amount of the miR-92 antagonist to the subject, and thereby treating or preventing the heart disease in the subject. Item 14: The miR-92 antagonist for use of any one of items 1 to 13, wherein the miR-92 antagonist is or comprises a nucleic acid.
Item 15: The miR-92 antagonist for use of item 14, wherein the nucleic acid is an antisense nucleic acid, preferably comprising a sequence that is at least partially complementary to miR-92.
Item 16: The miR-92 antagonist for use of item 14 or 15, wherein the nucleic acid is an oligonucleotide and comprises at least one locked nucleic acid (LNA) containing a 2' to 4' methylene bridge.
Item 17: The miR-92 antagonist for use of any one of items 14 to 16, wherein the nucleic acid comprises one or more T - deoxy-ribose nucleotides.
Item 18: The miR-92 antagonist for use of any one of items 14 to 17, wherein the nucleic acid further comprises one or more phosphorothioate modified nucleotides.
Item 19: The miR-92 antagonist for use of any one of items 14 to 18, wherein the nucleic acid comprises one or more modified nucleobases.
Item 20: The miR-92 antagonist for use of any one of items 14 to 19, wherein the modified nucleobase comprises one or two modified cytosines.
Item 21: The miR-92 antagonist for use of item 20, wherein the modified cytosine(s) comprise 5'- methylcytosine.
Item 22: The miR-92 antagonist for use of any one of items 14 to 21, wherein the nucleic acid has a 5' cap structure, 3' cap structure, or 5' and 3' cap structure.
Item 23: The miR-92 antagonist for use of any one of items 14 to 22, wherein the nucleic acid comprises one or more phosphorothioate linkages.
Item 24: The miR-92 antagonist for use of item 23, wherein the nucleic acid is fully phosphorothioate- linked.
Item 25: The miR-92 antagonist for use of any one of items 1 to 24, further comprising a pendent lipophilic group.
Item 26: A pharmaceutical composition comprising a therapeutically effective amount of the miR-92 antagonist recited in any one of items 14-25, or a pharmaceutically acceptable salt thereof, and a pharmaceutically-acceptable carrier or diluent.
Item 27: The pharmaceutical composition of item 26, wherein the pharmaceutically acceptable carrier comprises a colloidal dispersion system, macromolecular complex, nanocapsule, microsphere, bead, oil-in-water emulsion, micelle, mixed micelle, or liposome. Item 28: The pharmaceutical composition of item 26 or 27 for use in the treatment or prevention of a heart disease in a subject, wherein the heart disease is caused by or associated with a reduced ventricular elasticity and/or a diastolic dysfunction.
Item 29: The pharmaceutical composition for use of item 23, wherein the use is the use recited in any one of items 1 to 25.

### BRIEF DESCRIPTION OF THE FIGURES AND SEQUENCES

The figures show:
**Figure 1****:** shows treatment schedule with miR-92 LNA and control LNA in a model for HFpEF.
**Figure 2****:** shows results of echocardiographic measurements before treatment (baseline) and at week 4 (W4) week 7 (W7) and week 10 (W10). Shown is E/E' values (top graphs) and left atrial chamber echocardiographic measured area (LA area; bottom graphs).
**Figure 3****:** shows results of echocardiographic measurements at week 4, week 7 (W7) and week 10 (W10). Shown is E/E' values (top graphs) and left atrial chamber echocardiographic measured area (LA area; bottom graphs).
**Figure 4****:** shows results of histological measurements of the hearts at week 10. Shown is the fibrosis in percentage of the area.
**Figure 5****:** shows results of RNA measurements at week 10. Shown is the miR-92a-knockdown in whole blood (top left graph), kidney (top right graph), liver (bottom left graph) and heart (bottom right graph) as percentage of LNA-Co.
   **SEQ ID NO: 1** - pertains to the nucleic acid sequence of an antagomir against miR 92 and has the sequence: CAGGCCGGGACAAGUGCAAUA.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Antagonist of miR-92a prevents diastolic dysfunction in a model of HFpEF

To induce HFpEF mice were fed a high fat diet with 60% fat content (TD06414, Envigo) and additionally L-NAME (Sigma-Aldrich, 0.5 g/l) was added to their drinking water. Control mice received a normal Chow diet. No Ischemia was induced in the mice.

Echocardiographic measurements were performed at baseline (before HFpEF-induction), at week 4, 7 and 10 using a Vevo 3100 from Fujifilm Visualsonics.

Animals from the LNA-92a- and LNA-Control-groups received a weekly injection of the respective LNA in a concentration of 1.5 mg/kg BW starting two weeks after the HFpEF-induction.

At the harvest day, all animals underwent a catheter measurement to obtain PV-Loops (pressure-volume-loops) using a 1F catheter and a MPVS-Ultra system from Millar.

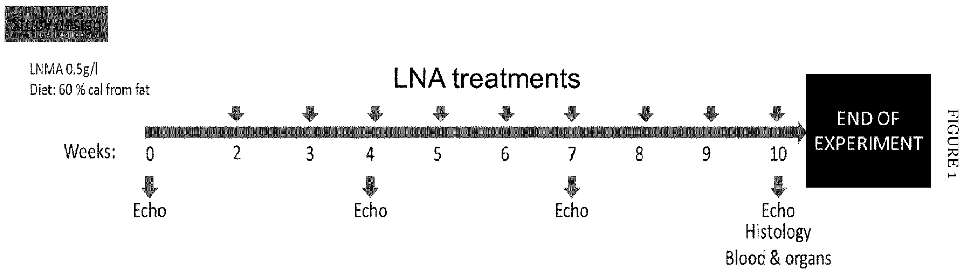

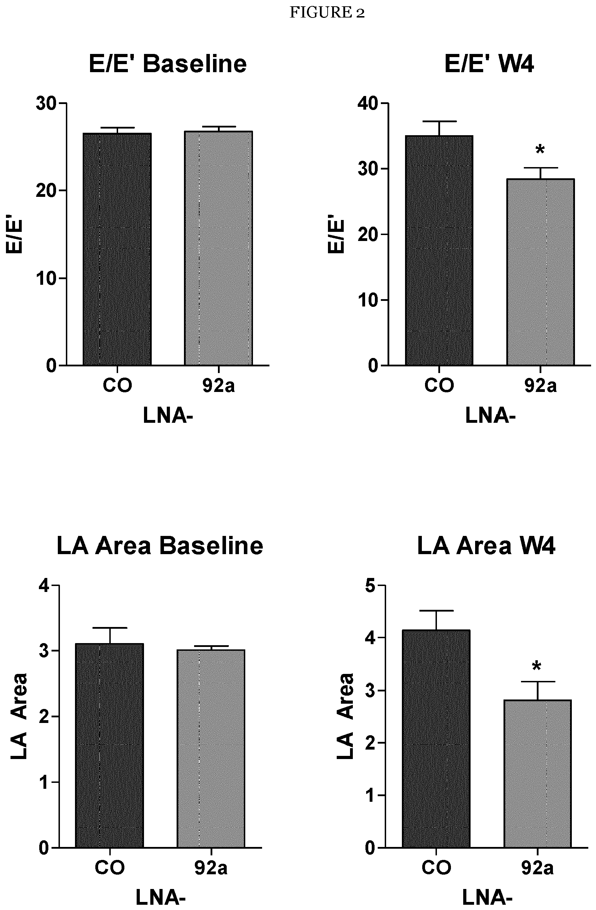

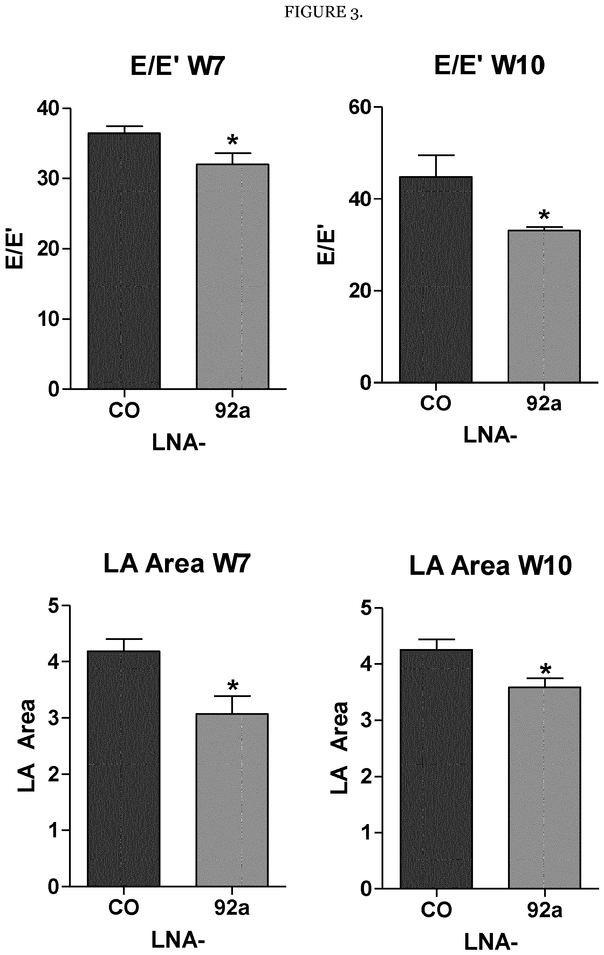

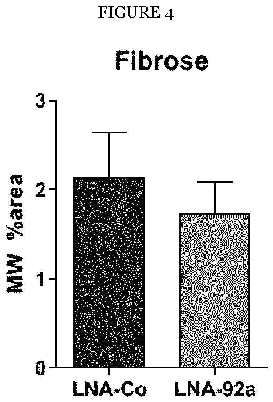

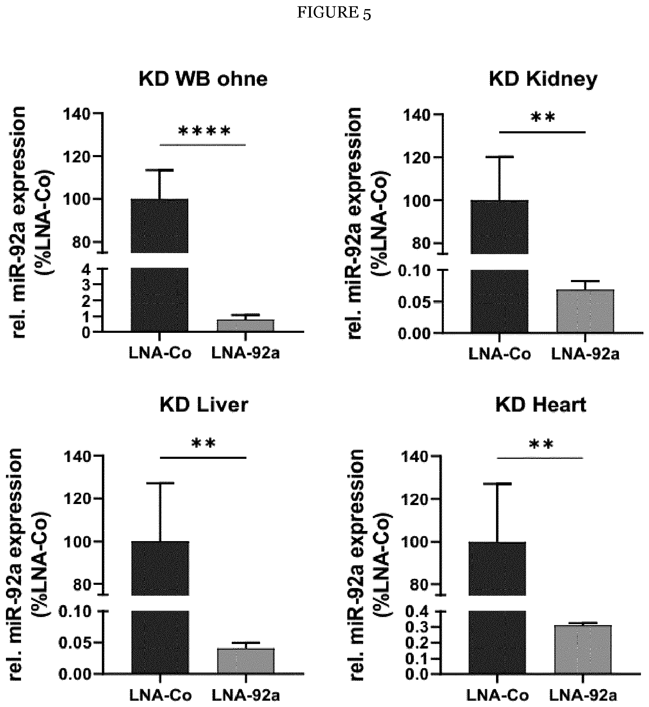

LNA-92a treatment was started at week 2 after initiation of LNMA/high fat diet treatment (Figure 1). Systolic and diastolic function was measured before, and at weeks 4, 7 and 10 by echocardiography ("Echo" in Figure 1). Efficiency of LNA-92a was demonstrated by the significant knockdown in wholeblood, kidney, liver and heart (Figure 5). It could be shown that LNA-92a treatment significantly prevents diastolic function as shown by the prevention of increase E/E' ratios (Figure 2 & 3). Increase in left atria area (also a marker of diastolic function) induced by the LNMA/high fat diet treatment was reduced by LNA-92a treatment (Figure 2 & 3). These results could also be further supported by a trend reduction of fibrosis by LNA-92a treatment (Figure 4).

In this model, no effect on left ventricular function or mass (not shown) and capillary density were observed.

Thus, these data suggest a novel mechanism by which LNA-92a specifically affects diastolic dysfunction. This effect is independent on the previously described effects on systolic function and is independent on patented effects of miR-92a on angiogenesis.

## Claims

1. **An miR-92 antagonist for use in the treatment or prevention of a heart disease** in a subject, wherein the heart disease is caused by or associated with or caused by a reduced ventricular elasticity and/or a diastolic dysfunction.

2. The miR-92 antagonist for use of claim 1, wherein the heart disease is Heart Failure with preserved Ejection Fraction (HFpEF).

3. The miR-92 antagonist for use of claim 2, wherein the heart disease is HFpEF irrespective of the presence or absence of coronary artery disease

4. The miR-92 antagonist for use of any one of claims 1 to 3, wherein the subject does not suffer from an ischemic heart disease.

5. The miR-92 antagonist for use of any one of claims 1 to 4, wherein the subject shows any one or a combination of:
(i) an abnormal measured exercise pulmonary artery pressure, such as an abnormal measured exercise pulmonary artery pressure of more than 30 mmHg; and/or
(ii) an abnormal measured exercise pulmonary capillary wedge pressure, such as an abnormal measured exercise pulmonary capillary wedge pressure of at least 25 mmHg; and/or
(iii) a normal measured resting pulmonary artery pressure or a normal measured resting pulmonary capillary wedge pressure; and/or
(iv) symptoms of left ventricular diastolic dysfunction.

6. The miR-92 antagonist for use of any one of claims 1 to 5, wherein the subject does not show symptoms of renal disease or cardiovascular disease.

7. The miR-92 antagonist for use of any one of claims 1 to 6, wherein the treatment or prevention comprises the administration of a therapeutically effective amount of the miR-92 antagonist to the subject, and thereby treating or preventing the heart disease in the subject.

8. The miR-92 antagonist for use of any one of claims 1 to 7, wherein the miR-92 antagonist is or comprises a nucleic acid.

9. The miR-92 antagonist for use of claim 8, wherein the nucleic acid is an antisense nucleic acid, preferably comprising a sequence that is at least partially complementary to miR-92.

10. The miR-92 antagonist for use of claim 8 or 9, wherein the nucleic acid is an oligonucleotide and comprises at least one locked nucleic acid (LNA) containing a 2' to 4' methylene bridge.

11. The miR-92 antagonist for use of any one of claims 8 to 10, wherein the nucleic acid comprises one or more modified nucleobases.

12. **A pharmaceutical composition** comprising a therapeutically effective amount of the miR-92 antagonist recited in any one of claims 1-10, or a pharmaceutically acceptable salt thereof, and a pharmaceutically acceptable carrier or diluent.

13. The pharmaceutical composition of claim 12, wherein the pharmaceutically acceptable carrier comprises a colloidal dispersion system, macromolecular complex, nanocapsule, microsphere, bead, oil-in-water emulsion, micelle, mixed micelle, or liposome.

14. The pharmaceutical composition of claim 12 or 13 for use in the treatment or prevention of a heart disease in a subject, wherein the heart disease is caused by or associated with a reduced ventricular elasticity and/or a diastolic dysfunction.

15. The pharmaceutical composition for use of claim 14, wherein the use is the use recited in any one of claims 1 to 11.
